(19) Europäisches Patentamt
European Patent Office
Office européen des brevets

(11) Veröffentlichungsnummer: **0 041 180**
**B1**

# (12) EUROPÄISCHE PATENTSCHRIFT

(45) Veröffentlichungstag der Patentschrift: **23.03.83**

(51) Int. Cl.³: **C 07 H 19/06**, C 07 H 19/04, C 12 P 19/38

(21) Anmeldenummer: **81103870.2**

(22) Anmeldetag: **20.05.81**

## (54) Verfahren zur Anreicherung von Nikkomycin-Gemischen.

(30) Priorität: **31.05.80 DE 3020722**

(43) Veröffentlichungstag der Anmeldung: **09.12.81 Patentblatt 81/49**

(45) Bekanntmachung des Hinweises auf die Patenterteilung: **23.03.83 Patentblatt 83/12**

(84) Benannte Vertragsstaaten: **AT BE CH DE FR GB IT LI NL**

(56) Entgegenhaltungen:
**EP-A-0 013 766**
**EP-A-0 022 964**
**US-A-4 158 608**

(73) Patentinhaber: **BAYER AG, Zentralbereich Patente, Marken und Lizenzen, D-5090 Leverkusen 1, Bayerwerk (DE)**

(72) Erfinder: **Moeschler, Heinrich-Ferdinand, Dr., Hahnenweg 8, D-5000 Koeln 80 (DE)**
Erfinder: **Gölker, Christian, Dr., Am Rohm 45, D-5600 Wuppertal 1 (DE)**
Erfinder: **Lange, Peter Michael, Dr., Walter-Flex-Strasse 9, D-5090 Leverkusen 1 (DE)**

EP 0 041 180 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Anreicherung von Nikkomycin-Gemischen

Die vorliegende Erfindung betrifft ein neues Verfahren zur Anreicherung von Nikkomycin-Gemischen.

Nikkomycin, seine Herstellung auf mikrobiologischem Weg mittels des Stammes Streptomyces tendae Ettlingen (CBS 354.75) und seine Verwendung als Pflanzenschutzmittel sind bekannt (vgl. deutsche Offenlegungsschrift 2 537 028, US-Patentschriften 4 046 881 und 4 158 608). Es stellte sich heraus, daß das derart gewonnene Nikkomycin ein Stoffgemisch aus sich ähnelnden Stoffen besteht, welche Nikkomycine genannt werden. Die bei der Fermentation und Aufarbeitung gewonnenen Nikkomycin-Gemische entsprechen in ihren Hauptbestandteilen der Formel I

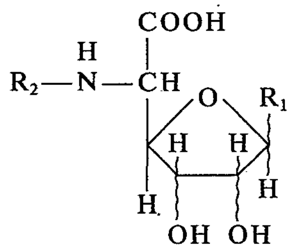

(I)

in der $R_1$ vor allem die Bedeutung

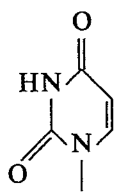

und

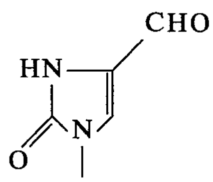

und $R_2$ vor allem die Bedeutung

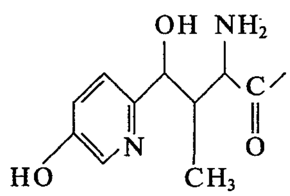

hat.

Nikkomycine sind wertvolle Mittel zur Bekämpfung von Pflanzenschädlingen und können beispielsweise als Insektizide, insbesondere Akarizide oder Fungizide verwendet werden.

Die Erfindung betrifft ein neues Verfahren zur Anreicherung der Nikkomycine, welche als Nikkomycin (X) und Nikkomycin (Z) bezeichnet werden. Nikkomycin (X) und Nikkomycin (Z) sowie ihre Gemische haben sich als besonders bedeutsam erwiesen.

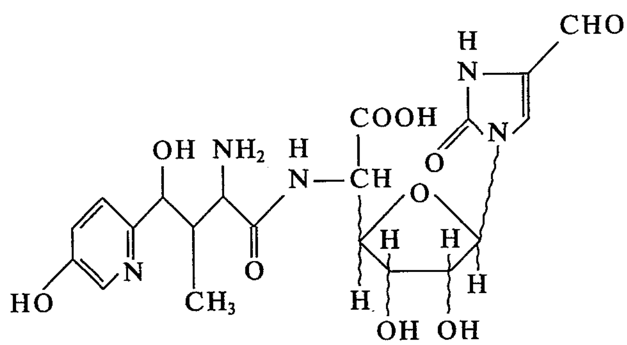

Nikkomycin (X)

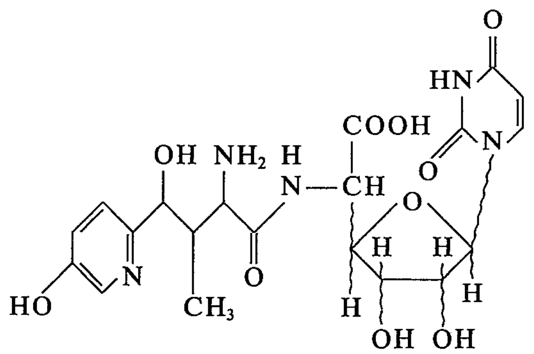

Nikkomycin (Z)

Aus den obengenannten Schutzrechten ist es bereits bekanntgeworden, Nikkomycin dadurch anzureichern, daß man in einer ersten Stufe das bei der Fermentation erhaltene Kulturfiltrat mit Essigsäure auf pH 4,0 ansäuert und mit einem saueren Ionenaustauscher in seiner Neutralform behandelt und anschließend mit verdünntem Ammoniak eluiert und die so gewonnene Lösung aufkonzentriert, anschließend in einer zweiten Stufe das Nikkomycin enthaltende Konzentrat mit Essigsäure auf pH 4,0 ansäuert und mit einem weiteren schwach saueren Ionenaustauscher in seiner Neutralform behandelt und anschließend mit verdünntem Ammoniak eluiert und die so gewonnene Lösung aufkonzentriert, anschließend in einer dritten Stufe das Nikkomycin enthaltende Konzentrat mit Essigsäure auf pH 4,0 ansäuert und mit SP-Sephadex C-25$^R$ (Warenzeichen der Firma Pharmacia Fine Chemicals, Upsala, Schweden) behandelt und anschließend mit Pyridin-Acetat-Puffer eluiert und die so gewonnene Lösung aufkonzentriert, schließlich in einer vierten Stufe mit Bio-Gel P-2$^R$ (Warenzeichen der Bio-Rad-Laboratories, Richmond, Cal. USA) behandelt und mit Wasser eluiert und die so gewonnene Lösung lyophilisiert.

Das Verfahren ist auf Grund seiner Vierstufigkeit sehr aufwendig und für technische Zwecke nicht voll befriedigend.

Es wurde nun gefunden, daß man das Nikkomycin-Komponentenpaar (X) und (Z) in einfacher Weise und hoher Selektivität anreichern kann, indem man Rohnikkomycin oder eine Nikkomycin enthaltende Kulturbrühe mit einem basischen Ionenaustauscher behandelt, das (X)- und (Z)-Gemisch mit einer Säure eluiert und das erhaltene Produkt nach den üblichen Methoden isoliert.

Es ist als ausgesprochen überraschend zu bezeichnen, daß bei der erfindungsgemäßen Adsorption des (X)- und (Z)-Gemisches am basischen Ionenaustauscher das Nikkomycin-Komponentenpaar (X) und (Z) unverändert bleibt, obwohl bereits seit längerer Zeit bekannt ist, daß Polyoxine und somit auch Nikkomycine ausgesprochen alkalilabil sind. Es war nach dem Stand der Technik zu erwarten, daß Nikkomycin am basischen Ionenaustauscher hydrolysiert wird und somit das gewünschte Produkt nicht erhalten wird.

Wie bereits oben dargelegt, erreicht man gemäß dem neuen Verfahren eine sehr gute Anreicherung der Nikkomycine (X) und (Z). Der Grad der Anreicherung ist selbstverständlich abhängig von der Art des Ausgangsmaterials und der Art und Menge des verwendeten basischen Ionenaustauschers sowie des Elutionsmittels.

Art und Beschaffenheit des erfindungsgemäß einsetzbaren Rohnikkomycins sind weitestgehend unkritisch. Das Rohnikkomycin kann in vorgereinigter Form oder in einer nicht vorgereinigten, bei der mikrobiellen Erzeugung anfallenden Form, nach seiner Isolierung oder aber in Form einer Lösung, wie sie bei der Aufarbeitung der Kulturbrühe anfällt, eingesetzt werden. Möglichst soll das Rohnikkomycin (wenn es in isolierter Form vorliegt) wenigstens 0,5%, vorzugsweise wenigstens 10% und ganz besonders bevorzugt 20 bis 30% des Gemisches der Nikkomycine (X) und (Z) enthalten (Gewichtsprozente bezogen auf Trockenmasse). Beispielsweise kann das gemäß der deutschen Offenlegungsschrift 2 537 028 oder den entsprechenden US-Patentschriften 4 046 881 und 4 158 608 erhaltene Nikkomycin verwendet werden.

In einer bevorzugten Ausführungsform der Erfindung wird das nach der Fermentation erhältliche Kulturfiltrat (vgl. die obengenannten Schutzrechte) ohne weitere Aufarbeitung direkt verwendet. Diese Verfahrensweise zeichnet sich durch eine besondere Einfachheit aus.

In einer weiteren bevorzugten Ausführungsform der Erfindung wird in einem ersten Schritt das nach der Fermentation erhaltene Kulturfiltrat mit einem sauren Ionenaustauscher behandelt. Bei anschließender Elution mit Ammoniak wird eine Rohnikkomycin-Lösung gewonnen, welche für eine Anreicherung der Nikkomycine (X) und (Z) nach dem erfindungsgemäßen Verfahren besonders gut geeignet ist.

Zur Durchführung des erfindungsgemäßen Verfahrens wird, falls das Rohnikkomycin nicht bereits als Lösung vorliegt oder ein Kulturfiltrat eingesetzt wird, das Rohnikkomycin in vorzugsweise entmineralisiertem oder destilliertem Wasser gelöst. Die Konzentration der Lösung ist unkritisch und wird lediglich durch die Löslichkeit des Nikkomycins einerseits und dem Wunsch nach einer leicht handhabbaren Flüssigkeitsmenge (Größe der Apparaturen) andererseits begrenzt. Die Lösung des Rohnikkomycins wird, falls nicht ein entsprechender pH-Wert bereits vorliegt, durch Säurezusatz auf

einen pH-Wert von 4 bis 7, vorzugsweise von 6 bis 7 gebracht. Hierzu können alle üblichen anorganischen (z. B. HCl) und vorzugsweise organischen Säuren, insbesondere niedere aliphatische Carbonsäuren, wie Essigsäure oder Propionsäure verwendet werden, welche Nikkomycin nicht angreifen. Bevorzugt ist Essigsäure.

Die wäßrige Lösung (welche auch organische Lösungsmittel, wie Methanol enthalten kann) wird in üblicher Weise mit dem basischen Ionenaustauscher in Kontakt gebracht (z. B. durch Aufgeben auf eine Säule oder durch Einrühren in einen Kessel). Die günstigste Menge an Ionenaustauscher ist abhängig von der eingesetzten Lösung und von der Art des Ionenaustauschers und kann nach üblichen Methoden leicht ermittelt werden.

Für die Durchführung des erfindungsgemäßen Verfahrens kommen alle üblichen basischen Ionenaustauscher in Frage. Beispielhaft seien aufgeführt:

Basische makroporöse oder gelförmige mit Divinylbenzol vernetzte Polystyrolharze, die teilweise oder vollständig mit primären, sekundären, tertiären oder quartären Stickstoffgruppen substituiert sind. Ebenso sind auch makroporöse und gelförmige Ionenaustauscher verwendbar, die sich von vernetztem Polyacrylamid ableiten.

Weiterhin können basische gelförmige oder makroporöse Ionenaustauscher auf Acryl- oder Methacrylsäureesterbasis, die mit z. B. Dimethylaminopropylamin umamidiert wurden, eingesetzt werden. Der funktionelle Stickstoff kann primär, sekundär, tertiär, quartär oder als Gemisch diesen Gruppierungen vorliegen. Besonders bevorzugt wird als schwach basischer Ionenaustauscher ein makroporöses aminomethyliertes Polystyrol, welches mit ca. 6% Divinylbenzol vernetzt ist (vgl. deutsche Patentschrift 2 418 976 und US-Patentschrift 3 989 650).

Erfindungsgemäß können als basische Ionenaustauscher auch basische gelförmige Ionenaustauscher auf Dextranbasis eingesetzt werden.

Als spezielle Beispiele für basische Ionenaustauscher seien genannt: Lewatit MP 500 (Warenzeichen der BAYER AG, Leverkusen, Deutschland [BRD]), Dowex MSA-1 (Warenzeichen der Dow Chemicals, USA) sowie DEAE-Sephadex A-25 und QAE-Sephadex A 25 (Warenzeichen der Pharmacia, Uppsala, Schweden).

Vorzugsweise wird anschließend der beladene Ionenaustauscher ein oder mehrere Male mit Wasser gewaschen.

Zur Elution des Nikkomycin-Komponentenpaares (X) + (Z) vom basischen Ionenaustauscher eignen sich verdünnte Lösungen von niedrigen aliphatischen Carbonsäuren, z. B. Essigsäure, die auf einfache Weise, z. B. durch Abdampfen oder Abschleppen mit anderen geeigneten Lösungsmitteln leicht zu entfernen sind, ohne daß dabei der pH-Wert in Richtung eines sauren Milieus so stark verändert wird, daß Hydrolyse des Komponentenpaares (X) + (Z) eintritt. Die Konzentration liegt vorzugsweise im Bereich von 0,1 – 10%, insbesondere bei 1 – 5% (Gew.-%).

Die Elution des Nikkomycin (X) und (Z) Gemisches kann nach den allgemein üblichen Methoden, z. B. durch einfaches Ausrühren mit Lösungen steigender Säurekonzentration oder durch Gradientenelution über eine Säule erfolgen.

Beim Ausrühren des mit dem Nikkomycin-Gemisch (X) + (Z) beladenen basischen Ionenaustauschers mit Lösungen steigender Säurekonzentration erhält man in den ersten Fraktionen eine bevorzugte Anreicherung von Nikkomycin (Z), während später bei höheren Säurekonzentrationen hauptsächlich Nikkomycin (X) angereichert wird.

Das Mischungsverhältnis von Nikkomycin (X) und (Z) kann also je nach Wunsch durch Abtrennen der jeweils zutreffenden Fraktionen stark beeinflußt werden.

Die Isolierung des Gemisches der Nikkomycine (X) und (Z) aus dem Eluat erfolgt nach den in der Biochemie allgemein üblichen Methoden, z. B. durch Verdampfen des Lösungsmittels, vorzugsweise unter vermindertem Druck oder durch Gefriertrocknung.

Der Vollständigkeit halber sei darauf hingewiesen, daß das erfindungsgemäße Verfahren auch mehrfach nacheinander durchgeführt werden kann.

Wie bereits oben dargelegt wurde, kann man in bevorzugten Ausführungsformen der Erfindung direkt von einem Kulturfiltrat der mikrobiellen Erzeugung ausgehen oder auch eine angereicherte Rohnikkomycin-Lösung einsetzen, wobei zur Anreicherung in einer vorgeschalteten Stufe ein Kulturfiltrat mit einem sauren Ionenaustauscher behandelt wird, bei nachfolgender Elution mit einer schwachen Base. Dieser Schritt soll in folgendem näher erläutert werden.

Das bei der mikrobiellen Erzeugung des Nikkomycin in bekannter Weise erhaltene Kulturfiltrat wird auf einen pH-Wert von 2 bis 5, vorzugsweise 3,5 bis 4,5, insbesondere 4 durch Säurezugabe eingestellt. Als Säuren sind hierbei solche Säuren geeignet, welche die obigen pH-Werte einzustellen vermögen. Vorzugsweise werden niedere aliphatische Carbonsäuren, insbesondere Essigsäure verwendet.

Diese Lösung wird nach allgemein üblichen Methoden mit einem sauren Ionenaustauscher behandelt.

Man kann die Nikkomycine (X) und (Z) z. B. durch einfaches Ausrühren mit dem Ionenaustauscher oder durch Auftragen oder Durchfluß der Nikkomycin-Lösung durch eine mit Ionenaustauscher gefüllte Säule binden.

Als saure Ionenaustauscher eignen sich vorzugsweise die üblichen makroporösen oder gelförmigen Ionenaustauscher aus mit Divinylbenzol vernetzten Polystyrolharzen mit Sulfonsäuregruppierungen

z. B. Lewatit SC 104 (Warenzeichen der BAYER AG, Leverkusen, Deutschland [BRD]) und Dowex 50 WX 4 (Warenzeichen der Dow Chemicals USA).

Vorzugsweise wird der beladene Ionenaustauscher ein oder mehrere Male mit Wasser gewaschen.

Zur Elution des Nikkomycin-Komponentenpaares (X) und (Z) von sauren Ionenaustauschern eignen sich schwache Basen, beispielsweise verdünntes Ammoniak. Die Konzentration liegt hierbei vorzugsweise im Bereich von 0,01 N – 0,1 N, insbesondere bei 0,04 – 0,06 N.

Das Eluat oder eine Lösung der aus dem Eluat isolierten Rohnikkomycins wird, wie oben beschrieben, auf den geforderten pH-Wert gebracht und mit dem basischen Ionenaustauscher behandelt.

Das erfindungsgemäße Verfahren soll durch die folgenden Beispiele erläutert werden. Die Ausbeuten wurden jeweils durch HPLC (High Pressure Liquid Chromatograpy) bestimmt. Die Ausbeuteangaben beziehen sich jeweils auf den Gehalt der Mischung im eingesetzten Rohnikkomycin. Das Endprodukt wurde aus der Lösung jeweils durch Lyophilisierung gewonnen. Der in den Beispielen 3 bis 6 eingesetzte schwach basische Ionenaustauscher ist ein makroporöses aminomethyliertes Polystyrol, welches mit 6% Divinylbenzol vernetzt ist (vgl. deutsche Patentschrift 2 418 976 und US-Patentschrift 3 989 650). Der Ionenaustauscher wurde vor der Verwendung jeweils mit Wasser gewaschen und mit Essigsäure auf pH 7,0 eingestellt. Wo nicht anders angegeben, beziehen sich die %-Angaben auf Gew.-%.

## Beispiel 1

### Elution mit Gradient

100 g Rohnikkomycin [ca. 15% (X) + (Z)] werden in 400 ml entmineralisiertem Wasser gelöst und der pH-Wert der Lösung mit Essigsäure auf pH 6,5 – 7,0 eingestellt. Diese Lösung wird zu 1 kg des in 1600 ml entmineralisierten Wasser suspendierten basischen Ionenaustauschers Lewatit MP 500 (mit Divinylbenzol vernetztes Polystyrolharz mit quartären Aminogruppen) in der Acetatform (mit $H_2O$ gewaschen) gegeben, wobei man den pH-Wert der Lösung bei pH 6,5 – 7,0 hält und 1 Stunde rührt. Es wird abgesaugt und der Ionenaustauscher in eine Säule (60 cm × 7,5 cm ∅) gefüllt und mit entmineralisiertem Wasser gewaschen, bis das Eluat farblos ist. Die Säule wird mit einem linearen Gradienten aus 4,5 Ltr. entmineralisiertem Wasser und 4,5 Ltr. 10%ige Essigsäure eluiert. Man erhält ca. 60 – 70%iges Nikkomycin (X) + (Z) in einer Ausbeute von 65%.

## Beispiel 2

### Stufenweise Elution im Batch-Verfahren

1 kg Rohnikkomycin [ca. 15% (X) + (Z)] wird in 4 Ltr. entmineralisiertem Wasser gelöst und der pH-Wert der Lösung mit Essigsäure auf pH 6,5 – 7,0 eingestellt. Diese Lösung wird zu 10 kg des in 16 Ltr. entmineralisierten Wasser suspendierten basischen Ionenaustauschers Lewatit MP 500 (mit Divinylbenzol vernetztes Polystyrolharz mit quartären Aminogruppen) in der Acetatform (mit Wasser gewaschen) gegeben, wobei man den pH-Wert der Lösung bei pH 6,5 – 7,0 hält und 1 Stunde rührt. Danach wird abgesaugt und das beladene Austauscherharz dreimal je 15 Minuten mit 10 Ltr. Wasser ausgerührt. Das gewaschene Ionenaustauscherharz wird daraufhin stufenweise jeweils 1/2 Stunde mit je 10 Ltr. Essigsäure steigender Konzentration (1%, 2%, 3%, 4%, 5%, 10%) ausgerührt, wobei der pH-Wert durch Zugabe von Essigsäure während der jeweiligen Ausrührstufe konstant gehalten wird. Man erhält ein 50 – 70%iges Nikkomycin (X) + (Z) mit einer Ausbeute von 70%.

## Beispiel 3

### Elution mit Gradient

100 g Rohnikkomycin [ca. 20% (X) + (Z)] werden in 400 ml entmineralisiertem Wasser gelöst und der pH-Wert der Lösung mit Essigsäure auf pH 6,5 – 7,0 eingestellt. Diese Lösung wird zu 1 kg des in 1600 ml entmineralisierten Wasser suspendierten schwach basischen Ionenaustauschers (gewaschen und mit Essigsäure auf pH 7,0 eingestellt) gegeben, wobei man den pH-Wert der Lösung durch Zugabe von Essigsäure bei pH 6,5 – 7,0 hält und 1 Stunde rührt. Es wird abgesaugt und der Ionenaustauscher in eine Säule (60 cm × 7,5 cm ∅) gefüllt und mit entmineralisiertem Wasser gewaschen, bis das Eluat farblos ist. Die Säule wird mit einem linearen Gradienten aus 4,5 Ltr. entmineralisiertem Wasser und 4,5 Ltr. 10%ige Essigsäure eluiert. Man erhält ca. 70 – 75%iges Nikkomycin (X) + (Z) in einer Ausbeute von 65%.

## Beispiel 4

### Stufenweise Elution im Batch-Verfahren

1 kg Rohnikkomycin [ca. 15% (X) + (Z)] wird in 4 Ltr. entmineralisiertem $H_2O$ gelöst und der pH-Wert der Lösung durch Zugabe von Essigsäure auf pH 6,5 – 7,0 eingestellt. Diese Lösung wird zu 10 kg des in 16 Ltr. entmineralisiertem Wasser suspendierten schwach basischen Ionenaustauschers (gewaschen und mit Essigsäure auf pH 7,0 eingestellt) gegeben, wobei man den pH-Wert der Lösung durch Zugabe von Essigsäure bei 6,5 – 7,0 hält und 1 Stunde rührt. Danach wird abgesaugt und das beladene Austauscherharz dreimal je 15 Minuten mit 10 Ltr. Wasser ausgerührt. Das gewaschene Ionenaustauscherharz wird daraufhin stufenweise jeweils 1/2 Stunde mit je 10 Ltr. Essigsäure steigender Konzentration (1%, 2%, 3%, 4%, 5%, 10%) ausgerührt, wobei der pH-Wert durch Zugabe von Essigsäure während der jeweiligen Ausrührungsstufe konstant gehalten wird. Man erhält ein bis zu 70%iges Nikkomycin (X) + (Z) mit 80%iger Ausbeute.

## Beispiel 5

### Elution mit Gradient

5 kg Rohnikkomycin (15 – 30%) werden in 20 Ltr. entmineralisiertem Wasser gelöst und der pH-Wert mit Essigsäure auf 6,5 – 7,0 eingestellt. In einer Vorlage mit Rührer werden 80 Ltr. entmineralisiertes Wasser und 50 kg des schwach basischen Ionenaustauschers (gewaschen mit Essigsäure auf pH 7,0 eingestellt) vorgelegt. Die Rohnikkomycin-Lösung wird dazugegeben und die Mischung 60 Minuten lang gerührt. Der pH-Wert wird durch Zugabe der entsprechenden Menge an Essigsäure auf einen pH-Wert von 6,5 – 7,0 gehalten. Der beladene Ionenaustauscher wird zweimal mit je 100 Ltr. entmineralisiertem Wasser gewaschen. Danach wird der Ionenaustauscher in eine konische Glassäule eingefüllt. Die Säule wird eluiert mit einem linearen Gradienten, hergestellt aus 600 Ltr. entmineralisiertem Wasser und 600 Ltr. 10%iger Essigsäure. Der Säulenvorlauf wird verworfen. Die Elution des Nikkomycins wird anhand des pH-Wertes und der Leitfähigkeit verfolgt.

Der Reinheitsgrad beträgt ca. 90% mit einer Ausbeute von 70 – 80%.

## Beispiel 6

### Stufenweise Elution im Batch-Verfahren

5 kg Rohnikkomycin [15 – 30% an (X) + (z)] werden in 20 Ltr. entmineralisiertem Wasser gelöst und der pH-Wert mit Essigsäure auf 6,5 – 7,0 eingestellt. In einer Vorlage mit Rührer werden 80 Ltr. entmineralisiertem Wasser und 50 kg des schwach basischen Ionenaustauschers (gewaschen und mit Essigsäure auf pH 7,0 eingestellt) vorgelegt und die Rohnikkomycin-Lösung eingerührt. Es wird 60 60 Minuten lang gerührt. Der pH-Wert wird durch Zugabe von Essigsäure auf einen Wert von 6,5 – 7,0 gehalten. Der beladene Ionenaustauscher wird dreimal mit je 100 Ltr. entmineralisiertem Wasser gewaschen.

Der Ionenaustauscher wird darauf stufenweise mit je 50 Ltr. Essigsäure steigender Konzentration (1, 3, 4 und 10%) unter Kontrolle und Konstanthalten des pH-Wertes eluiert. Der Reinheitsgrad beträgt ca. 90% mit einer Ausbeute von 70 – 80%.

## Beispiel 7

### Herstellung von Rohnikkomycin aus einer Kulturbrühe durch Adsorption mit einem sauren Ionenaustauscher

2000 l Fermentationsbrühe (vgl. z. B. Deutsche Offenlegungsschrift 2 537 028 und US-Patentschriften 4 046 881 und 4 158 608) werden über einen Separator geschleudert und das Zentrifugat mit Essigsäure auf einen pH-Wert von 4,0 eingestellt. Es wird nochmals über einen Separator geklärt, wobei man einschließlich des zur Verdrängung in der Trommel notwendigen Spülwassers ca. 2400 l Filtrat erhält. Dieses Filtrat wird mit 750 l eines Kationenaustauschers Lewatit SC 104 (auf Polystyrol-Basis) in der $Na^+$-Form 30 Minuten lang gerührt. Der Überstand wird abgesaugt und das Harz mit 1250 l entmineralisiertem Wasser durch Rühren ausgewaschen. Zur Elution wird der Ionenaustauscher 10 Minuten lang mit 0,01 N Ammoniaklösung (1125 l) ausgerührt. Das Eluat wird mittels Saugdüsen abgesaugt. Der pH-Wert des Eluates muß zwischen 6 und 6,8 liegen. Danach wird der Ionenaustauscher mit 0,05 N Ammoniaklösung (1800 l) 30 Minuten lang ausgerührt. Der Überstand wird ebenfalls abgesaugt. Danach wird nochmals 10 Minuten lang mit 0,1 N Ammoniaklösung (360 l)

gerührt. Die Hauptmenge der Nikkomycinaktivität wird im 2. Eluat gefunden. Dieses wird durch Verdampfen des Lösungsmittels unter vermindertem Druck konzentriert und anschließend lyophilisiert. Das so erhaltene Rohnikkomycin wird in den vorstehenden Beispielen 1 bis 6 eingesetzt.

Beispiel 8

10 Liter Kulturbrühe (nach Abtrennung des Maycels und Einstellen des pH-Wertes mit Essigsäure auf 4,0) werden mit 1 kg basischem Ionenaustauscher [aminomethyliertes Polystyrol (makroporös) mit 6% Divinylbenzol vernetzt], der vorher mit Essigsäure gewaschen und auf pH 7,0 eingestellt wurde, unter Rühren versetzt.

Der pH-Wert der Lösung wird durch Zugabe von Essigsäure bei pH 6,5—7,0 gehalten und die Suspension wird eine Stunde gerührt.

Danach wird abgesaugt und das beladene Austauscherharz dreimal je 15 Minuten mit 1 Liter Wasser ausgerührt. Das gewaschene Ionenaustauscherharz wird daraufhin stufenweise mit je 1,5 Litern Essigsäure steigender Konzentration (1%, 2%, 3%, 4%, 5%, 10%) ausgerührt, wobei der pH-Wert der Lösung durch Zugabe von Essigsäure während der jeweiligen Ausrührstufe konstant gehalten wird. Man erhält ein 40—60%iges Nikkomycin mit einer Ausbeute von 85%.

**Patentansprüche**

1. Verfahren zur Anreicherung der Nikkomycine (X) und (Z) aus einem Kulturfiltrat der mikrobiellen Nikkomycin-Erzeugung oder aus Rohnikkomycin, dadurch gekennzeichnet, daß man das Kulturfiltrat oder das Rohnikkomycin, gegebenenfalls nach einer Vorreinigung durch Adsorption an einem sauren Ionenaustauscher und Elution mit einer schwachen Base und sich gegegenenfalls anschließende Isolierung, in wäßriger Lösung mit einem schwach basischen Ionenaustauscher behandelt und mit einer Säure eluiert und das Nikkomycin (X) und (Z) Gemisch nach den üblichen Methoden isoliert.

2. Verfahren gemäß Anspruch 1, dadurch gekennzeichnet, daß man das Kulturfiltrat der mikrobiellen Nikkomycin-Erzeugung durch Adsorption an einem sauren Ionenaustauscher und Elution, mit gegebenenfalls sich anschließender Isolierung, vorreinigt und dieses Rohnikkomycin in wäßriger Lösung mit einem schwach basischen Ionenaustauscher behandelt und mit einer Säure eluiert und das Nikkomycin (X) und (Z) Gemisch nach den üblichen Methoden isoliert.

3. Verfahren gemäß Anspruch 1, dadurch gekennzeichnet, daß man das Kulturfiltrat der mikrobiellen Nikkomycin-Erzeugung ohne Vorreinigung mit einem schwach basischen Ionenaustauscher behandelt und mit einer Säure eluiert und nach den üblichen Methoden isoliert.

4. Verfahren gemäß den Ansprüchen 1 und 2, dadurch gekennzeichnet, daß man als einen sauren Ionenaustauscher einen makroporösen oder gelförmigen Ionenaustauscher aus mit Divinylbenzol vernetzten Polystyrolharzen mit Sulfonsäuregruppierungen einsetzt.

5. Verfahren, gemäß den Ansprüchen 1 bis 4, dadurch gekennzeichnet, daß man als basische Ionenaustauscher makroporöse oder gelförmige mit Divinylbenzol vernetzte Polystyrolharze, die teilweise oder vollständig mit primären, sekundären, tertiären oder quartären Stickstoffgruppen substituiert sind oder makroporöse und gelförmige Ionenaustauscher, die sich von vernetztem Polyacrylamid ableiten oder basische gelförmige oder makroporöse Ionenaustauscher auf Acryl- oder Methacrylsäureesterbasis, die mit Dimethylaminopropylamin umamidiert wurden, und in welchen der funktionelle Stickstoff primär, sekundär, tertiär, quartär oder als Gemisch dieser Gruppierungen vorliegen kann, einsetzt.

6. Verfahren gemäß Ansprüchen 1 bis 5, dadurch gekennzeichnet, daß man als schwach basischen Ionenaustauscher ein makroporöses aminomethyliertes Polystyrol, welches mit ca. 6% Divinylbenzol vernetzt ist, einsetzt.

7. Verfahren, gemäß den Ansprüchen 1, 2, 4, 5 und 6, dadurch gekennzeichnet, daß man als schwache Base zur Elution vom sauren Ionenaustauscher verdünntes Ammoniak verwendet.

8. Verfahren gemäß den Ansprüchen 1 bis 7, dadurch gekennzeichnet, daß man die Nikkomycin enthaltende wäßrige Lösung vor der Adsorption am schwach basischen Ionenaustauscher mit Essigsäure auf einen pH-Wert zwischen 4 und 7 bringt.

9. Verfahren gemäß den Ansprüchen 1 bis 8, dadurch gekennzeichnet, daß man zur Elution des Nikkomycin (X) und (Z) Gemisches vom basischen Ionenaustauscher Essigsäure mit steigender Säurekonzentration verwendet.

**Claims**

1. Process for concentrating nikkomicins (X) and (Z) from a culture filtrate obtained from the production of nikkomicin by a microbial route or from crude nikkomicin, characterised in that the culture filtrate or the crude nikkomicin, if appropriate after prepurification by absorption onto an acid

ion exchanger and elution with a weak base and, if necessary, subsequent isolation, are treated, in aqueous solution, with a weakly basic ion exchanger and eluted with an acid and the mixture of nikkomicin (X) and nikkomicin (Z) is isolated by the customary methods.

2. Process according to Claim 1, characterised in that the culture filtrate from the production of nikkomicin by a microbial route is prepurified by absorption onto an acid ion exchanger and elution, if necessary with subsequent isolation, and this crude nikkomicin is treated, in aqueous solution, with a weakly basic ion exchanger and eluted with an acid and the mixture of nikkomicin (X) and nikkomicin (Z) is isolated by the customary methods.

3. Process according to Claim 1, characterised in that the culture filtrate from the production of nikkomicin by a microbial route is treated, without prepurification, with a weakly basic ion exchanger and eluted with an acid and the nikkomicin is isolated by the customary methods.

4. Process according to Claims 1 and 2, characterised in that a macroporous or gel-like ion exchanger of polystyrene resins which are crosslinked with divinylbenzene and contain sulphonic acid groupings is employed as a relatively acid ion exchanger.

5. Process according to Claims 1 to 4, characterised in that macroporous or gel-like polystyrene resins which are crosslinked with divinylbenzene and are partly or completely substituted by primary, secondary, tertiary or quaternary nitrogen groups, or macroporous or gel-like ion exchangers which are derived from crosslinked polyacrylamide, or basic gel-like or macroporous ion exchangers which are based on acrylates or methacrylates and have been trans-amidated with dimethylaminopropylamine, and in which the functional nitrogen can be in the form of a primary grouping, secondary grouping, tertiary grouping or quaternary grouping or as a mixture of these groupings, are employed as the basic ion exchangers.

6. Process according to Claims 1 to 5, characterised in that a macroporous amino-methylated polystyrene crosslinked with about 6% of divenylbenzene is employed as the weakly basic ion exchanger.

7. Process according to Claims 1, 2, 4, 5 and 6, characterised in that dilute ammonia is used as the weak base for eluting the acid ion exchanger.

8. Process according to Claims 1 to 7, characterised in that the aqueous solution containing nikkomicin is brought to a pH value of between 4 and 7 with acetic acid, before adsorption onto the weakly basic ion exchanger.

9. Process according to Claims 1 to 8, characterised in that acetic acid of increasing concentration is used for eluting the mixture of nikkomicin (X) and nikkomicin (Z) from the basic ion exchanger.

## Revendications

1. Procédé pour concentrer les nikkomycines (X) et (Z) dans un filtrat de culture de production microbienne de nikkomycine ou à partir de nikkomycine brute, caractérisé en ce qu'on traite en solution aqueuse avec un échangeur d'ions faiblement basiques, le filtrat de culture ou la nikkomycine brute, éventuellement après une purification préalable par adsorption sur un échangeur d'ions acides et élution avec une base faible suivie éventuellement d'un isolement, et on élue avec un acide et on isole le mélange de nikkomycines (X) et (Z) par les procédés classiques.

2. Procédé suivant la revendication 1, caractérisé en ce qu'on purifie préalablement le filtrat de culture de production microbienne de nikkomycine par adsorption sur un échangeur d'ions acides et élution, avec éventuellement isolement subséquent, et on traite cette nikkomycine brute en solution aqueuse avec un échangeur d'ions faiblement basiques et on l'élue avec un acide, et on isole le mélange de nikkomycines (X) et (Z) par des opérations classiques.

3. Procédé suivant la revendication 1, caractérisé en ce qu'on traite le filtrat de culture de production microbienne de nikkomycine sans purification préalable avec un échangeur d'ions faiblement basiques et on effectue une élution avec un acide et l'isolement par des procédés classiques.

4. Procédé suivant les revendications 1 et 2, caractérosé en ce qu'on utilise comme échangeur d'ions acides un échangeur ionique macroporeux ou sous la forme de gel obtenu à partir de résines polystyrène réticulées au divinylbenzène, avec des groupements acide sulfonique.

5. Procédé suivant les revendications 1 à 4, caractérisé en ce qu'on utilise comme échangeurs d'ions basiques des résines de polystyrène macroporeuses ou sous forme de gel, réticulées au divinylbenzène, qui sont partiellement ou totalement substituées avec des groupes azotés primaires, secondaires, tertiaires ou quaternaires ou des échangeurs ioniques macroporeux et sous forme de gel qui dérivent de Polyacrylamide réticulé ou des échangeurs ioniques basiques sous forme de gel ou macroporeux à base d'ester d'acide acrylique ou d'acide méthacrylique, qui ont été transamidés avec la diméthylaminopropylamine, et dans lesquels l'azote fonctionnel peut être présent sous la forme primaire, secondaire, tertiaire, quaternaire ou sous la forme d'un mélange de ces groupements.

6. Procédé suivant les revendications 1à 5, caractérisé en ce qu'on utilise comme échangeur d'ions faiblement basiques un polystyrène aminométhylé macroporeux qui est réticulé avec environ 6% de divinylbenzène.

7. Procédé suivant les revendications 1, 2, 4, 5 et 6, caractérisé en ce qu'on utilise de l'ammoniaque

diluée comme base faible pour l'élution de l'échangeur d'ions acides.

8. Procédé suivant les revendications 1 à 7, caractérisé en ce qu'on ajuste entre 4 et 7 avec de l'acide acétique le pH de la solution aqueuse contenant la nikkomycine avant l'adsorption sur l'échangeur ionique faiblement basique.

9. Procédé suivant les revendications 1 à 8, caractérisé en ce qu'on utilise de l'acide acétique à concentration croissante pour l'élution du mélange de nikkomycines (X) et (Z) de l'échangeur d'ions basiques.